(19) Europäisches Patentamt
European Patent Office
Office européen des brevets

(11) **EP 4 068 305 A1**

(12) **EUROPEAN PATENT APPLICATION**

(43) Date of publication:
**05.10.2022 Bulletin 2022/40**

(21) Application number: **22164949.4**

(22) Date of filing: **29.03.2022**

(51) International Patent Classification (IPC):
**G16H 50/30** (2018.01)     **A61B 5/00** (2006.01)

(52) Cooperative Patent Classification (CPC):
**G16H 50/30; A61B 5/7275; A61B 5/742; A61B 5/7475**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2021   IT 202100007922**
**11.11.2021   IT 202100028643**

(71) Applicant: **Riatlas S.r.l.**
**84060 Novi Velia (SA) (IT)**

(72) Inventors:
• **ROMANELLI, Luca**
**20133 Milano (IT)**
• **ORCIUOLI, Francesco**
**84085 Mercato San Severino (SA) (IT)**
• **FENZA, Giuseppe**
**84010 Maiori (SA) (IT)**
• **ORCIUOLI, Francesco**
**83025 Montoro (AV) (IT)**

(74) Representative: **De Ros, Alberto et al**
**Studio Legale Bird & Bird**
**Via Borgogna, 8**
**20122 Milano (IT)**

(54) **METHOD FOR DISPLAYING ON A SCREEN OF A COMPUTERIZED APPARATUS A TEMPORAL TREND OF A STATE OF HEALTH OF A PATIENT AND COMPUTERIZED APPARATUS**

(57)     The method serves to display and advantageously modify a display on a screen of a computerized apparatus a temporal trend of a state of health (P10) of a patient; the computerized apparatus stores health data related to a set of different health parameters (P1, P2) of said patient; for each parameter (P1, P2) a plurality of absolute values of said parameter at different temporal instants is stored; the method comprises the steps of: choosing the state of health (P10) among said different states of health (P10, P20, P100); determining a level of the state of health (P10) and a set of health parameters (P1, P2) among said plurality of health parameters (P1, P2, P3, P4, P5, P6, P7) associated with the state of health (P10) taking into account the level; choosing a scale to represent said state of health (P10), said scale being numerical in particular discrete numerical; converting the absolute values (100, 200) of the parameters of said set (P1, P2) into relative values (110, 210) according to said scale; for each temporal instant, combining the relative values of the parameters of said set (P1, P2) according to a mathematical formula (F) obtaining a plurality of aggregate values (PA) at different temporal instants; and displaying a temporal graph (400, 500) of said aggregate values on the screen.

Fig. 5

EP 4 068 305 A1

**Description**

**FIELD OF THE INVENTION**

The present invention relates to a method for displaying on a screen of a computerized apparatus a temporal trend of at least one state of health of a patient and computerized apparatus adapted to implement such a method.

**BACKGROUND ART**

The "state of health" of a patient can be indicated by the value of various health parameters.

For example, the values of blood parameters obtained from a person's blood examination together indicate a certain "state of health" of this person, but in a non-synthetic format.

The "perceptive capacities" of a person indicate, as a whole, a certain "state of health" of this person (which has nothing to do with the "state of health" mentioned in the previous paragraph). There are five human senses (sight, hearing, smell, touch and taste) and if measurements are taken on a person in relation to the various senses, their "perceptive capacities" are quantified. It is well known that a person's "perceptive capacities" vary over time; for example, sight and hearing decline with age, but not in the same way for all people.

A person's "perceptive capacities" can be divided and grouped. For example, "relevant perceptive capacities for driving a vehicle", i.e., vision and hearing, can be identified and grouped together. The "relevant perceptive capacities for driving a vehicle" also indicate, as a whole, a certain "state of health" (which is different from the "state of health" referred to in the previous paragraph, although related).

The various possible parameters of a person's health are very different.

The values of different health parameters are measured with different units of measure. For example, sight is measured in diopters and hearing is measured in decibels.

**SUMMARY**

From patent document US2012/116180A1, it is known a system and method for providing a state of health of a patient which receives as input data related to health parameters of a patient, transforms and combines them with each other generating a "health score" of the patient to monitor the state of health of a patient for example in a hospital or nursing home.

However, the Applicant is not aware of any method for aggregating different health parameters of a person and providing a concise indication of a "state of health" of this person in which the health parameters are organized or can be organized on several distinct hierarchical levels; indeed, despite what has been explained in the preceding paragraphs, even the idea of having health parameters organized on several distinct hierarchical levels is not known.

Even less well known is the display on a screen of a computerized apparatus of a temporal trend of a patient's "state of health"; a "state of health" which is to be understood as an aggregation of several different health parameters of this person and which can be at the same and/or different levels.

The general object of the present invention is to solve such a problem of displaying a temporal trend of a state of health related to different and nonhomogeneous health parameters, i.e., inhomogeneous in which the health parameters are organized or can be organized on several distinct hierarchical levels.

A further object of the present invention is to provide a method for modifying the display of a temporal trend of a state of health related to different and nonhomogeneous health parameters which modifies the display at least qualitatively (it could be an "increase" in quality or a "decrease" in quality, which does not mean a worsening of the display) and possibly also quantitatively.

This general object and other more specific objects are reached thanks to what is expressed in the appended claims that form an integral part of the present description.

**LIST OF FIGURES**

The present invention shall become more readily apparent from the detailed description that follows to be considered together with the accompanying drawings in which:

Fig. 1 shows two tables storing absolute values of two health parameters, respectively,
Fig. 2 shows two tables storing relative values of the same two health parameters of Fig. 1, respectively,
Fig. 3 shows a table storing aggregated values of the two health parameters of
Fig. 1 and Fig. 2,

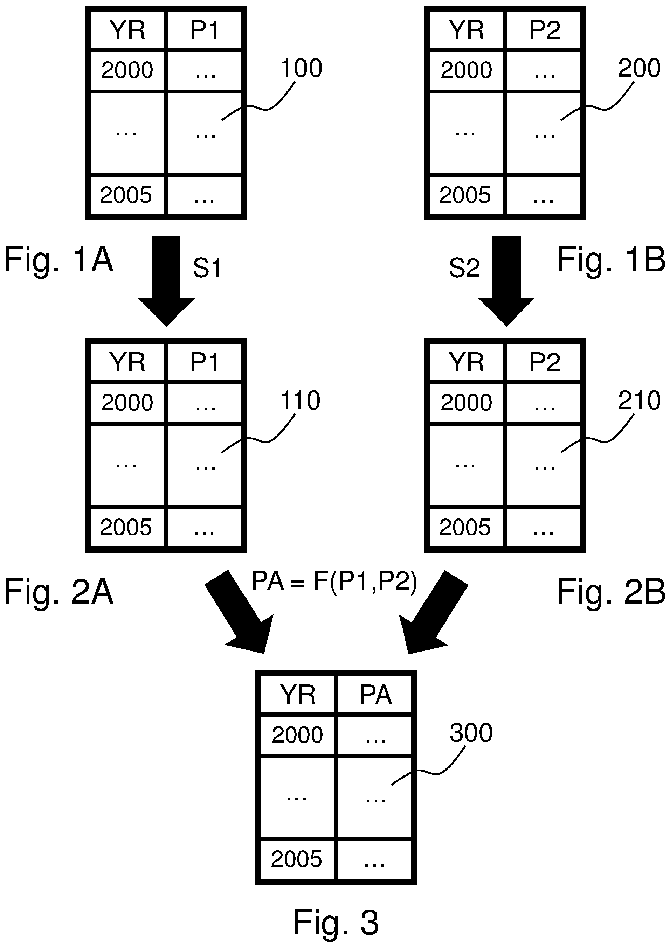

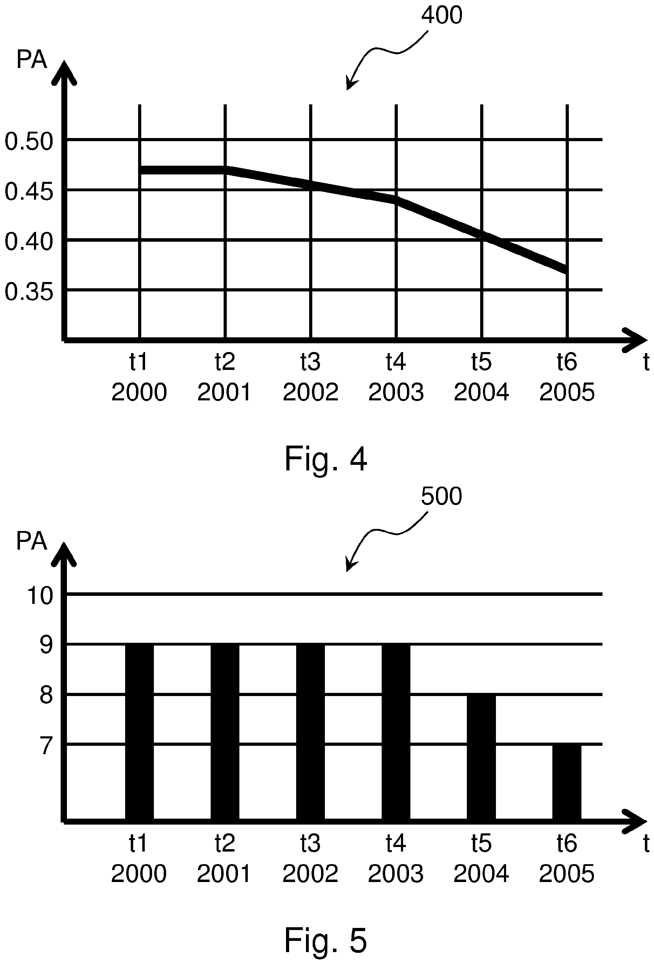

Fig. 4 shows a first example embodiment of a temporal graph related to the aggregated values of the table of Fig. 3, and

Fig. 5 shows a second example embodiment of a temporal graph related to the aggregated values of the table of Fig. 3,

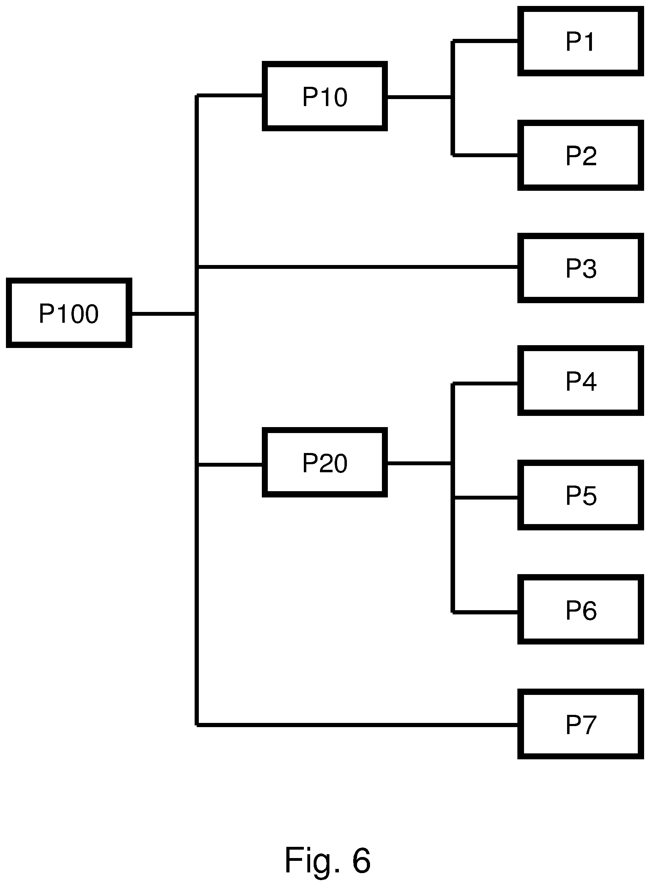

Fig. 6 shows an example of a possible aggregation of health parameters on several levels,

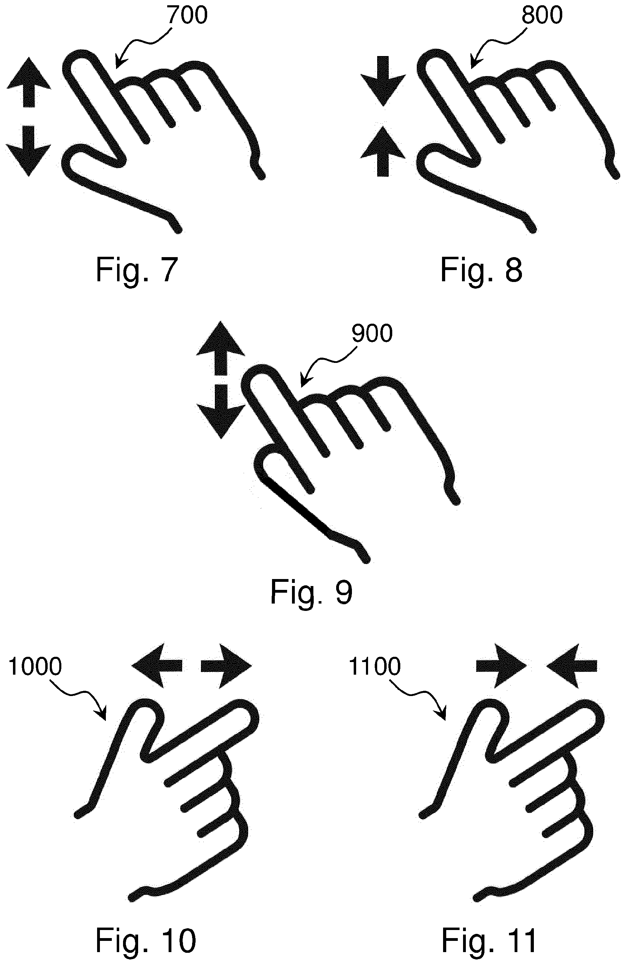

Fig. 7 shows a vertical "zoom-in" gesture,
Fig. 8 shows a vertical "zoom-out" gesture,
Fig. 9 shows a vertical "scroll" gesture,
Fig. 10 shows a horizontal "zoom-in" gesture, and
Fig. 11 shows a horizontal "zoom-out" gesture.

As it can be easily understood, there are various ways of practically implementing the present invention which is defined in its main advantageous aspects in the appended claims and is not limited either to the following detailed description or to the appended drawings.

## DETAILED DESCRIPTION

The present invention will be described below with reference to a patient's state of health related to his "perceptual capabilities" because the relevant examples are easy to understand, in particular the example relating to "perceptive capabilities relevant for driving vehicles". This easy understanding should not, however, lead one to think that the invention is obvious. In fact, the idea of aggregating inhomogeneous health parameters is by no means trivial, especially if one considers it in the context of medical science, and is particularly advantageous as the number of aggregating parameters increases.

The idea of aggregating inhomogeneous health parameters is the basis for visualising the temporal trend of at least one state of health of a patient according to the present invention, which is effective because it is simple and easy to understand.

The present invention is useful not only for doctors, but also for paramedics and patients.

As is known already been mentioned above, the human senses are five: sight, hearing, smell, touch and taste.

"Perceptive capacities" can be the subject of a patient's first "state of health" (if the person is considered to have a disease or disorder related to their perceptive capacities), and refer to all five senses.

The "relevant perceptive capacities for driving a vehicle" can be the subject of a patient's second "state of health", and can relate, for example, to vision and hearing.

The "relevant perceptive capacities for nutrition" can be the subject of a third "state of health" of a patient, and can refer, for example, to taste and smell; but could additionally refer to touch and sight.

In the following we will refer to the same person as "patient".

Table 100 of Fig. 1A stores the absolute values of the parameter P1, i.e., vision, of a person in the years 2000 to 2005, assuming they have carried out one measurement in "diopters" per year. The absolute values are, for example, as follows:

| | |
|---|---|
| 2000 | 10 diopters |
| 2001 | 10 diopters |
| 2002 | 10 diopters |
| 2003 | 9.5 diopters |
| 2004 | 9 diopters |
| 2005 | 8.5 diopters |

We assume that they are all acceptable values for driving a vehicle, except for the 2005 value.

Table 200 of Fig. 1B stores the absolute values of the parameter P2, i.e., hearing, of a person in the years 2000 to 2005, assuming they have carried out one measurement in "decibels" per year. The absolute values are, for example, as follows:

| | |
|---|---|
| 2000 | 10 decibels |
| 2001 | 10 decibels |
| 2002 | 15 decibels |
| 2003 | 15 decibels |
| 2004 | 20 decibels |
| 2005 | 25 decibels |

We assume that they are all acceptable values for driving a vehicle.

Table 110 of Fig. 2A stores the relative values of parameter P1, which are derived from the conversion of the absolute values of table 100 of Fig. 1A using a scale from 0 to 1, where 1 indicates optimal vision (i.e., 10 diopters) and the formula P1/10; the relative values are as follows:

| | |
|---|---|
| 2000 | 1.0 % |
| 2001 | 1.0 % |
| 2002 | 1.0 % |
| 2003 | 0.95 % |
| 2004 | 0.9 % |
| 2005 | 0.85 % |

Table 210 of Fig. 2B stores the relative values of parameter P2, which are derived from the conversion of the absolute values of table 200 of Fig. 1B using a scale from 0 to 1, where 1 indicates optimal vision (i.e., 0 decibels) and the formula 1-P2/50; the relative values are as follows:

| | |
|---|---|
| 2000 | 0.8 % |
| 2001 | 0.8 % |
| 2002 | 0.7 % |
| 2003 | 0.7 % |
| 2004 | 0.6 % |
| 2005 | 0.5 % |

Table 300 of Fig. 3 stores aggregated PA values obtained, for each year considered, by combining the relative values of parameters P1 and P2 according to a mathematical formula F; the formula is as follows:

$$PA(year) = (0.7 * P1r(year) + 0.3 * P2r(year)) / 2$$

The aggregate values are as follows:

| | |
|---|---|
| 2000 | 0.47 |
| 2001 | 0.47 |
| 2002 | 0.455 |
| 2003 | 0.4375 |
| 2004 | 0.405 |
| 2005 | 0.3725 |

The acceptability of these aggregate values will be considered later.

Such aggregated values (which can be between 0 and 1) could be processed by discretizing them on a scale of, for example, 0 to 20; the aggregated and discretized values are as follows:

| | |
|---|---|
| 2000 | 9 |
| 2001 | 9 |
| 2002 | 9 |
| 2003 | 9 |
| 2004 | 8 |
| 2005 | 7 |

The acceptability of these aggregated and discretized values will be considered later.

Fig. 4 shows a temporal graph 400 related to the above aggregated values as it could be displayed on a screen of a computerized apparatus, e.g., a desktop or laptop or tablet or smartphone.

Fig. 5 shows a temporal graph 500 related to the above aggregated and discretized values as it could be displayed on a screen of a computerized apparatus, e.g., a desktop or laptop or tablet or smartphone.

It is evident how effective the display of the state of health of "relevant perceptive capacities for driving a vehicle" is, even if it is derived from several nonhomogeneous health parameters, i.e., vision and hearing, and the relative development over time.

Furthermore, the "aggregated view" also facilitates an "aggregated assessment" of the state of health. For example, if first the vision parameter is considered alone, the conclusion can be reached that the person can drive without problems up to (and including) 2004, if the hearing parameter in then considered alone, the conclusion can be reached that the person could drive without problems up to (and including) 2005 and then, overall, it could be deduced that the person could drive without problems up to (and including) 2004. On the other hand, if the aggregation (and the related graphs 400 and 500) are considered, it could be deduce that the perceptive capacities as a whole have drastically reduced after 2003 and therefore driving could already be problematic in 2004; this message is even more evident from graph 500 which corresponds to an "aggregated and discretized" view of the state of health.

Of course, the foregoing (and the relative formulae) are only examples which should be regarded as non-limiting to the present invention.

Examples could have been given of the display of other states of health, e.g., with reference to what was explained above, the state of health related to "relevant perceptive capacities for nutrition" or the state of health related to all "perceptive capacities". It is clear that for the same person, various "states of health" can be considered by choosing different health parameters.

A significant example of "health parameters" consists of blood parameters.

A significant example of "health parameters" consists of "ICF codes" (International Classification of Functioning, Disability and Health) with the relative "domains" and "qualifiers". Typically, for an individual patient values will only be available from a group of a few ICF codes, and the set considered for display purposes could be a subset of the group; it can also make sense to define several subsets within the same group.

However, according to the present invention, the "health parameters" which can be aggregated for display purposes can be completely inhomogeneous and obtained by means of completely different examinations.

Two aspects are important for the practical usefulness of the present invention: the formulas for converting the absolute values of the health parameters to the relative values of the health parameters, and the combination formula; the combination is typically an "aggregation function", a term used in the field of computing.

The invention will be explained below in its generality; the preceding example can be helpful in understanding what is expressed below.

As already mentioned, the method according to the present invention serves to display on a screen of a computerized apparatus a temporal trend of at least one state of health of a patient.

The method envisages that the computerized apparatus stores health data related to a set of different health parameters of the patient; such data will be acquired and entered into the apparatus prior to the display, typically well before the display; it is possible, indeed likely, that the apparatus will store other data related to the patient, in particular other health data related to the patient in addition to the set considered herein. Specifically, for each health parameter of the set considered here, a plurality of absolute values of the parameter is stored at different temporal instants; in the example described above, reference was made to one datum per year, but the period could be completely different (one datum per month, one datum per week, one datum per day, one datum per hour, etc.) and the data could also simply refer to repeated surveys/measurements which are not strictly periodical. As can be seen from the example described, it is important that for each instant considered (which, as mentioned, can have a certain duration) there is an absolute value of each parameter of the set considered.

The method generally comprises the steps of:

A) choosing the state of health (see P10 in Fig. 6) between different states of health (see P10, P20, P100 in Fig. 6);
B) determining a level (or hierarchical level) of the chosen state of health (see P10 in Fig. 6) and a set of health parameters (see P1, P2 in Fig. 6) among the plurality of health parameters (see P1, P2, P3, P4, P5, P6, P7 in Fig. 6) associated with the chosen state of health (see P10 in Fig. 6) taking into account its level;
C) choosing a scale to represent the state of health under consideration;
D) converting the absolute values of the parameters of the set into relative values according to such a scale;
E) for each temporal instant, combining the relative values of the parameters of the set according to a mathematical formula, obtaining a plurality of values aggregated in different temporal instants (which are the same temporal instants in which the parameters to be combined are defined) - it is therefore an operation to be repeated identically for each temporal instant; and
F) displaying a temporal graph of the aggregated values on the screen.

As will be better understood when describing Fig. 6, according to this example, there are three states of health in total (P10, P20 and P100), organized on two distinct levels, and there are seven health parameters in total (P1, P2, P3, P4, P5, P6 and P7). The state P10 is associated with parameters P1 and P2. The state P20 is associated with parameters P4, P5 and P6. The state P100 can be considered as being associated with all parameters P1 to P7, but also as being associated with lower-level states P10 and P20; in particular in the example of Fig. 6, the state P100 can be considered to be associated both with lower-level states (and thus indirectly with health parameters) and directly with health parameters (in Fig. 6, P3 and P7). The determination referred to in B can be made, in particular, on the basis of knowledge of a predetermined hierarchical structure (see for example Fig. 6). The scale referred to in step C of the method is numeric (e.g., between 0 and 1 or between 0 and 100), particularly discrete numeric (e.g., it could be an integer between 0 and 9).

The conversion referred to in step D of the method can also envisage that not all values of the scale are used, e.g., the scale goes from 0 to 9 but only values from 0 to 4 are chosen to be used.

The mathematical formula referred to in step E of the method can vary widely. As mentioned above, it is typically an "aggregation function", e.g., a maximum function (note that there is not only the"absolute maximum") or a minimum function (note that that there is not only the"absolute minimum") or an average function (e.g.,there is linear average, weighted average, geometric average) or a linear combination or a non-linear combination; in the case of a linear combination, the core of the combination formula are the multiplication coefficients. A good or optimal choice can depend on the application and/or the operator preferences.

Among the various possibilities, such a formula could also advantageously include a discretization operation (see the difference between graph 400 in Fig. 4 and graph 500 in Fig. 5) to be carried out after the aggregation operation.

The set of health parameters according to the present invention (i.e., its composition) can be selected by an operator from a plurality of patient health parameters during the use of the computerized apparatus for the display. Preferably, the set can be modified, for example as a function of a disease of the patient presumed by the operator. The set can correspond to a choice previously made by an operator and stored in the device; for example, if an operator has to assess a group of patients, he may be interested in the same set for all patients in the group.

One of the possible choices of the operator for the display is the temporal frame of interest; for example, a patient's data can be stored in the device between the year 2000 and the year 2020 and, at a certain point in time, the operator may only be interested in, for example, the last ten years, or the last three years, or the last year, or the first two years, etc.

The set of health parameters according to the present invention (i.e., its composition) can be selected from a plurality of possible health parameters by an installer prior to use of the computerized apparatus for the display. Preferably, the set can be modified, for example, as a function of an application of the device; for example, if the device is installed in a driving school, it could be set up so that it always considers only the health parameters related to "relevant perceptive capacities for driving vehicles".

The set of health parameters according to the present invention (i.e., its composition) can depend on health data of the patient stored in the computerized apparatus. In particular, it can depend on a patient disease estimated by the device in an automatic or quasi-automatic manner.

The above three possibilities for determining the composition of the set can be combined with one another.

The mathematical formula according to the present invention can be chosen by an operator while using the computerized apparatus for the display. Preferably, it can be modified.

The mathematical formula according to the present invention can be chosen by an installer prior to use of the computerized apparatus for display. Preferably, it can be modified.

The mathematical formula according to the present invention can depend on the set of health parameters considered, in particular the composition of the set.

The three possibilities mentioned above concerning the mathematical formula can be combined with one another.

According to the most typical applications of the present invention, the set of health parameters consists of a minimum of three to a maximum of twenty parameters, even more typically of a minimum of four to a maximum of eight parameters.

So far, the case of simply aggregated health parameters has been considered, e.g., parameters P1 and P2 aggregated for display purposes to form a "state of health" PA. But the present invention can also be applied to health parameters organized on several levels, in particular in a predetermined manner, as shown for example in Fig. 6. The parameters P1-P7 are first-level health parameters, parameters P10 and P20 are second-level health parameters and parameter P100 is a third-level health parameter.

The parameter P10 (which can be considered to correspond to the "state of health" PA of the previous example) can be displayed as an aggregation of the parameters P1 and P2 (all first level), the parameter P20 as an aggregation of the parameters P4 and P5 and P6 (all first level), the parameter P100 as an aggregation of the parameters P10 and P20 (second level) and P3 and P7 (first level) - the three combination formulae related to these three aggregations can be equal (a very typical case), but alternatively similar or different. To explain further, the parameter P1 can be "sight", the parameter P2 can be "hearing", the parameter P10 can be "relevant perceptive capacities for driving vehicles", the parameter P20 can be "relevant mental capacities for driving vehicles", and the parameter P100 can be "relevant human capacities for driving vehicles" (comprising both the physical and mental abilities); the parameters P3 and P7 can be other specific abilities relevant for driving. It should be borne in mind that the aggregation of several health parameters according to the present invention need not necessarily result in a "state of health" (which can also be considered a new "health parameter") having a clear meaning which can be easily expressed in words; in the preceding paragraph, examples with clear meanings have been given only to facilitate understanding.

Advantageously, according to the present invention, an display on the screen of a patient's "state of health" such as the one just described can be suitably modified, at least qualitatively and possibly also quantitatively.

According to a very common typology, visualisations on a screen of a computerized apparatus of a patient's "state of health", such as the examples just described, correspond to temporal graphs which develop according to a first parametric direction, generally vertical ("y-axis"), and according to a second temporal direction, generally horizontal ("x-axis").

The display can be modified, for example, by enlarging (so-called "zoom-in") on the vertical axis and/or the horizontal axis or reducing (so-called "zoom-out") on the vertical axis and/or the horizontal axis.

By virtue of "touchscreens", i.e., touch-sensitive screens, mainly used in devices called "tablets" and "smartphones", changes in enlargement and reduction can be achieved by gestures which the user of the device carries out by touching the screen. A well-known gesture is to place the fingertip of the thumb and the fingertip of the index finger next to the screen at a certain distance from each other and to vary this distance (i.e., bring the fingertips closer together or move them apart) while keeping the fingertips close to the screen.

Furthermore, it is known to change the display in other ways, e.g., by scrolling; scrolling can also be achieved by gestures.

According to the present invention, a method is described in which, for each temporal instant, values of various health parameters are combined according to a mathematical formula, resulting in a plurality of aggregated values.

In general, the method for modifying the display according to the present invention can also be applied to displays obtained in a different manner.

At the heart of the method of modifying the display is the "qualitative zoom" (as opposed to the traditional and well-known "quantitative zoom") achieved in particular by means of "gestures".

With non-limiting reference to the example graphs of Fig. 4 and Fig. 5 already described, the temporal interval of the display is from t1 (e.g., the year 2000) to t6 (e.g., the year 2005), and the values are displayed annually.

For both of these example graphs, the horizontal x-axis corresponds to the temporal axis and the vertical y-axis corresponds to the parameter axis.

The parameter PA in these graphs is deliberately generic and refers to a patient's "state of health". It could be any of the parameters discussed above with reference to Fig. 6 (or even a different one); but the most significant example to explain such a change in the display of the parameters on the screen is P20. In the following it is assumed that Fig. 4 and Fig. 5 refer to the parameter P20.

The change of display on the screen can be applied (in particular typically applies) to health parameters organized on two or more (three, four, five, six, ...) levels as shown for example in Fig. 6 in which there are three levels.

Before describing an embodiment of such a modification, some gestures will be described, well known in themselves, which can be used to implement the modification of displaying a "state of health" of the patient on the screen; the use of different gestures is not excluded at all.

Fig. 7 schematically shows a "zoom-in" or "vertical" enlargementgesture. The gesture consists of placing the fingertip of the thumb and the fingertip of the index finger next to a screen at a certain distance from each other and increasing this distance (i.e., moving the fingertips apart) while keeping the fingertips close to the screen. It is called "vertical" because the distancing develops essentially along the vertical direction.

Fig. 8 schematically shows a "zoom-out" or "vertical" reduction gesture. The gesture consists of placing the fingertip of the thumb and the fingertip of the index finger next to a screen at a certain distance from each other and decreasing this distance (i.e., bringing the fingertips closer together) while keeping the fingertips close to the screen. It is called "vertical" because the approach develops essentially along the vertical direction.

Fig. 10 schematically shows a "zoom-in" or "horizontal" enlargement gesture. The gesture consists of placing the fingertip of the thumb and the fingertip of the index finger next to a screen at a certain distance from each other and increasing this distance (i.e., moving the fingertips apart) while keeping the fingertips close to the screen. It is called "horizontal" because the distancing develops essentially along the horizontal direction.

Fig. 11 schematically shows a "zoom-out" or "horizontal" reduction gesture. The gesture consists of placing the fingertip of the thumb and the fingertip of the index finger next to a screen at a certain distance from each other and decreasing this distance (i.e., bringing the fingertips closer together) while keeping the fingertips close to the screen. It is called "horizontal" because the approach develops essentially along the horizontal direction.

If the direction of distancing or approach cannot be regarded as "essentially" vertical or horizontal, it may be necessary to consider what the "main" direction is (e.g., in relation to a 45° direction).

Fig. 9 schematically shows a "scroll" or "vertical" scrolling gesture. The gesture consists of placing the fingertip of the index finger next to a screen and moving it while keeping it close to the screen. It is called "vertical" because the movement develops essentially along the vertical direction. Similarly, a "scroll" or "horizontal" scrolling gesture can be defined.

The starting point for the following explanation is, for example, the parameter P20, understood as the "state of health" of a patient, displayed as shown in Fig. 4 on a screen of a computerized apparatus; the parameter P20 corresponds to the set of health parameters P4 and P5 and P6 of the patient aggregated together (through a certain formula); the initial display corresponds to a temporal graph 400 defining a first direction or parametric direction PA and a second direction or temporal direction t. In response to a "zoom-in" gesture according to the first direction (see for example gesture 700 in Fig. 7 - the important thing is that the gesture is such that it can be associated with the first direction, i.e., the parametric direction) or with a "zoom-out" gesture according to the first direction (see for example gesture 800 in Fig. 8 - the important thing is that the gesture is such that it can be associated with the first direction, i.e. the parametric direction) of a user of the device, a different temporal graph is displayed on the screen. It corresponds to a different set of patient health parameters; if there is more than one parameter in the different set, they are aggregated together (through a certain formula which is typically different from the one used for the initial display).

In response to the "zoom-in" gesture in the first direction, the set of different parameters comprises parameters of a lower hierarchical level. Starting from the display of P20, the "zoom-in" gesture leads, for example, to the display of P5 which is the "central" parameter in the lower hierarchy level; alternatively, it could lead to the display of P4 which is the first parameter in the lower hierarchy level or P6 which is the last parameter in the lower hierarchy level. In this example, the different set comprises only one health parameter (and therefore no aggregation is necessary) as the lowest level of the hierarchy.

In response to the "zoom-out" gesture in the first direction, the set of different parameters comprises parameters of a higher hierarchical level. Starting from the display of P20, the "zoom-out" gesture leads to the display of P100. The different set comprises a number of health parameters (P10, P3, P20 and P7) and therefore needs to be aggregated.

According to further embodiment examples, the different set of parameters can depend on the magnitude of the gesture. For example, considering Fig. 3, a wide parametric "zoom-in" gesture can pass from parameter P100 to parameter P2 or P3 or P5 or P7. For example, considering Fig. 6, a wide parametric "zoom-out" gesture can pass from parameter P5 to parameter P100.

Advantageously, it can be envisaged that the display according to the present invention comprises not only a temporal graph, but also additional indications.

For example, the abbreviation and/or name of the health parameter corresponding to the current display and possibly also the aggregated health parameters and possibly also the "combination formula" can appear on the screen; with reference to Fig. 4 and Fig. 5, instead of the generic symbol PA, the specific symbol P20 and possibly also P4, P5, P6 could be shown. It is understood that many additional indications (textual and graphic) could be useful to the user; they could be displayed permanently (according to default settings) or at the user's request.

In response to a "scrolling" gesture in the first direction (see for example gesture 900 in Fig. 9 - the important thing is that the gesture is such that it can be associated with the first direction, i.e. the parametric direction) of a user of the device, a different temporal graph is displayed on the screen. It corresponds to a different set of patient health parameters; if there is more than one parameter in the different set, they are aggregated together (through a certain formula which can be the same, similar or different from the one used for the initial display).

In response to the "scrolling" gesture in the first direction, the different set of parameters comprises parameters of the same hierarchical level.

According to some embodiment examples, the different set can depend on the direction of movement of the gesture and/or the width of the movement of the gesture.

For example, starting from the display of P20, the scroll gesture leads to the display of P10 whether the movement is upwards or downwards. For example, starting from the display of P5, the scroll gesture leads to the display of P4 if the movement is upwards and P6 if the movement is downwards. For example, starting from the display of P4, the "scroll" gesture with an upward movement can lead, depending on the implementation, to the display of P3 or P6 ("cyclic scroll" within the set can be mentioned). For example, a broad gesture can pass you from displaying P4 directly to displaying P6.

In response to a "zoom-in" gesture in the second direction (see for example gesture 1000 in Fig. 10 - the important thing is that the gesture is such that it can be associated with the second direction, i.e. the temporal direction) or with a "zoom-out" gesture according to the second direction (see for example gesture 1100 in Fig. 11 - the important thing is that the gesture is such that it can be associated with the second direction, i.e. the temporal direction) of a user of the device, the same temporal graph is displayed on the screen, but with a respectively enlarged or reduced temporal scale.

With reference to Fig. 4 or Fig. 5, a horizontal, i.e., temporal, zoom-in gesture can lead to the (enlarged) display of for example the same parameter PA of the temporal interval t1-t5 or t1-t4 or t1-t3 or t2-t6 or t2-t5 or t3-t6 or t2-t4 or t3-t5 or t4-t6 depending on the magnitude of the gesture and/or the position of the gesture. In response to a "scrolling" gesture in the second direction (there is no figure specifically representing this - it could be a gesture similar to that shown in Fig. 9 but rotated by 90°) of a user of the apparatus, a different temporal graph is displayed on the screen. It corresponds to a different temporal interval, but with the same temporal scale.

With reference to Fig. 4 or Fig. 5, a horizontal, i.e., temporal, scroll gesture, starting from the display of the parameter PA in the temporal interval t3-t4 can lead to the display of the same parameter PA in the temporal interval t2-t3 or t4-t5 (depending on the direction of movement) or, if the gesture is large, in the temporal interval t1-t2 or t5-t6 (depending on the direction of movement). As will be understood, the method (of displaying and/or display modification) according to the present invention can be successfully and advantageously implemented and incorporated into a computerized apparatus having a screen, a processor and memory, for example a desktop or a laptop or a tablet or a smartphone.

In this case, typically, a computer program is stored in memory, and the program is such that the method is carried out when executed by the processor.

Such a program be pre-installed in the device or installed when required.

There is also the possibility that the device is only used for display purposes (particular by virtue of its screen) and that the processing of health data is performed by another device, e.g., a "local server" or a "cloud server". In other words, the computerized device could only perform HMI functions, i.e., human-machine interface for both input and output.

Advantageously, the method according to the present invention is particularly well suited to be implemented in a computerized apparatus having a screen of the "touch-sensitive" type; in this case, some or all of the gestures for changing the display on the screen are detected by the screen itself. However, as is known, there are other ways of detecting gestures, for example through a video camera.

**Claims**

1. Method for displaying on a screen of a computerized apparatus a temporal trend of a state of health (P10) of a patient between different states of health (P10, P20, P100) of said patient,

   wherein said different states of health (P10, P20, P100) are organized on several levels in a predetermined manner,
   wherein said computerized apparatus stores health data related to a plurality of different health parameters (P1, P2, P3, P4, P5, P6, P7) of said patient underlying said different states of health (P10, P20, P100),
   wherein, for each parameter (P1, P2, P3, P4, P5, P6, P7), a plurality of absolute values of said parameter at different temporal instants is stored,
   the method comprises the steps of:

   choosing said state of health (P10) from among said different states of health (P10, P20, P100),
   determining a level of said state of health (P10) and a set of health parameters (P1, P2) among said plurality of health parameters (P1, P2, P3, P4, P5, P6, P7) associated with said state of health (P10) taking into account said determined level,
   choosing a scale to represent said state of health (P10), said scale being numeric, in particular discrete numeric;
   converting the absolute values (100, 200) of the parameters of said set (P1, P2) into relative values (110, 210) according to said scale;
   for each temporal instant, combining the relative values of the parameters of said set according to a mathematical formula (F) obtaining a plurality of aggregate values (P10) at different temporal instants; and
   displaying a temporal graph (400, 500) of said aggregate values on the screen.

2. Method according to claim 1, wherein said set of health parameters (P1, P2) is selected by an operator among a plurality of health parameters (P1, P2, P3, P4, P5, P6, P7) of the patient during the use of said computerized apparatus, and preferably it can be modified, for example according to a disease of the patient presumed by the operator.

3. Method according to claim 2, wherein said set of health parameters (P1, P2) corresponds to a choice previously made by an operator and stored in said computerized apparatus.

4. Method according to any one of claims 1 to 3, wherein said set of health parameters (P1, P2) depends on health data of said patient stored in said computerized apparatus, in particular on a disease of said patient estimated by said computerized apparatus.

5. Method according to any one of claims 1 to 4, wherein said mathematical formula (F) depends on said set of health parameters (P1, P2), in particular on the composition of said set.

6. Method according to any one of claims 1 to 5, wherein said set of health parameters (P1, P2) consists of a minimum of three to a maximum of twenty parameters, typically a minimum of four to a maximum of eight parameters.

7. Method for modifying a display on a screen of a computerized apparatus of a state of health (P10) of a patient, said display being obtained according to any one of claims 1 to 6,

  wherein said display corresponds to a temporal graph (400, 500) of a set of health parameters (P10, P20) of said patient aggregated together, said temporal graph (400, 500) defining a first direction or parametric direction and a second direction or temporal direction,
  wherein in response to a "zoom-in" gesture according to said first direction (700) or a "zoom-out" gesture according to said first direction (800) of a user of said computerized apparatus, a different temporal graph is displayed on the screen, wherein said different temporal graph corresponds to a different set (P100; P1, P2; P4, P5, P6) of health parameters of said patient.

8. Method according to claim 7,
wherein in response to said "zoom-in" gesture according to said first direction (700), said different set (P1, P2; P4, P5, P6) of parameters comprises parameters of a lower hierarchical level.

9. Method according to claim 7 or 8,
wherein in response to said "zoom-out" gesture according to said first direction (800), said different set (P100) of parameters comprises parameters of a higher hierarchical level.

10. Method according to any one of claims 7 to 9, wherein in response to a "scrolling" gesture according to said first direction (900) of a user of said computerized apparatus, a different temporal graph is displayed on the screen, wherein said different temporal graph corresponds to a different set (P20, P10) of health parameters of said patient.

11. Method according to claim 10,
wherein in response to said "scrolling" gesture according to said first direction (900), said different set (P20, P10) of parameters comprises parameters of identical hierarchical level.

12. Method according to any one of claims 7 to 11, wherein in response to a "zoom-in" gesture according to said second direction (1000) or a "zoom-out" gesture according to said second direction (1100) of a user of said computerized apparatus, the same temporal graph is displayed on the screen, but with an enlarged or reduced temporal scale.

13. Method according to claim 12, wherein in response to a "scrolling" gesture according to said second direction by a user of said computerized apparatus, a different temporal graph is displayed on the screen, wherein said different temporal graph corresponds to a different temporal interval with the same temporal scale.

14. Method according to any one of claims 7 to 13, wherein said screen is a "touch sensitive" type, and wherein any or all of said gestures are detected by said screen.

15. Computerized apparatus having a screen, a processor and memory, wherein a computer program is stored in said memory, and wherein said program is such as to implement the method according to any one of the preceding claims when executed by said processor.

Fig. 1A

Fig. 1B

S1

S2

Fig. 2A

Fig. 2B

PA = F(P1,P2)

Fig. 3

400

Fig. 4

500

Fig. 5

Fig. 6

Fig. 7

Fig. 8

Fig. 9

Fig. 10

Fig. 11

**EUROPEAN SEARCH REPORT**

Europäisches Patentamt
European Patent Office
Office européen des brevets

Application Number

EP 22 16 4949

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/126124 A1 (SCHECHTER ALAN M [US]) 29 May 2008 (2008-05-29) * paragraphs [0012] – [0054] * | 1-6,15 | INV. G16H50/30 A61B5/00 |
| X | US 2012/116180 A1 (ROTHMAN MICHAEL J [US] ET AL) 10 May 2012 (2012-05-10) | 1,15 | |
| Y | * paragraphs [0070] – [0144]; figures 7,9 * | 7-14 | |
| A | WO 2020/132826 A1 (SHENZHEN MINDRAY BIOMEDICAL ELECTRONICS CO LTD [CN] ET AL.) 2 July 2020 (2020-07-02) * abstract * | 1-15 | |
| Y | US 2015/025405 A1 (VAIRAVAN SRINIVASAN [US] ET AL) 22 January 2015 (2015-01-22) * paragraphs [0005] – [0007], [0071] – [0072]; figure 8 * | 7-14 | |
| Y | US 2008/214904 A1 (SAEED MOHAMMED [US] ET AL) 4 September 2008 (2008-09-04) * paragraphs [0037], [0043]; figures 3,6,7 * | 7-14 | TECHNICAL FIELDS SEARCHED (IPC) G16H A61B |

The present search report has been drawn up for all claims

| Place of search | Date of completion of the search | Examiner |
|---|---|---|
| Munich | 5 August 2022 | Rivera Pons, Carlos |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 4949

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2022

| Patent document cited in search report | | Publication date | Patent family member(s) | | Publication date |
|---|---|---|---|---|---|
| US 2008126124 | A1 | 29-05-2008 | NONE | | |
| US 2012116180 | A1 | 10-05-2012 | AU | 2007308078 A1 | 17-04-2008 |
| | | | CA | 2666379 A1 | 17-04-2008 |
| | | | EP | 2073690 A2 | 01-07-2009 |
| | | | US | 2009105550 A1 | 23-04-2009 |
| | | | US | 2012116180 A1 | 10-05-2012 |
| | | | WO | 2008045577 A2 | 17-04-2008 |
| WO 2020132826 | A1 | 02-07-2020 | CN | 112996426 A | 18-06-2021 |
| | | | WO | 2020132826 A1 | 02-07-2020 |
| US 2015025405 | A1 | 22-01-2015 | CN | 104115150 A | 22-10-2014 |
| | | | EP | 2815343 A2 | 24-12-2014 |
| | | | JP | 6215845 B2 | 18-10-2017 |
| | | | JP | 6541738 B2 | 10-07-2019 |
| | | | JP | 6734430 B2 | 05-08-2020 |
| | | | JP | 2015513724 A | 14-05-2015 |
| | | | JP | 2018014131 A | 25-01-2018 |
| | | | JP | 2019169158 A | 03-10-2019 |
| | | | RU | 2014137469 A | 10-04-2016 |
| | | | US | 2015025405 A1 | 22-01-2015 |
| | | | WO | 2013121374 A2 | 22-08-2013 |
| US 2008214904 | A1 | 04-09-2008 | AT | 492208 T | 15-01-2011 |
| | | | CN | 101203172 A | 18-06-2008 |
| | | | EP | 1903932 A1 | 02-04-2008 |
| | | | JP | 5584413 B2 | 03-09-2014 |
| | | | JP | 2008543478 A | 04-12-2008 |
| | | | US | 2008214904 A1 | 04-09-2008 |
| | | | WO | 2006136972 A1 | 28-12-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012116180 A1